# EUROPEAN PATENT SPECIFICATION

(11) **EP 0 535 225 B1**
(45) Date of publication and mention of the grant of the patent: **16.07.1997**
(21) Application number: 90901902.8
(22) Date of filing: 19.01.1990
(51) Int. Cl.: B08B 9/46

(54) **METHOD FOR DISCRIMINATING RECOVERED CONTAINERS**
VERFAHREN ZUR SORTIERUNG VON WIEDERVERWENDBAREN BEHÄLTERN
PROCEDE DE TRIAGE DE CONTENEURS RECUPERES

(30) Priority: 19.01.1989 JP 18733/89
(43) Date of publication of application: 07.04.1993
(73) Proprietor: THE COCA-COLA COMPANY, Atlanta, Georgia 30313 (US)
(72) Inventor: OSAKADA, Kunio, Kawasaki-shi Kanagawa 215 (JP)
(74) Representative: Leale, Robin George
(86) International application number: PCT/JP90/00059
(87) International publication number: WO 90/07990

(56) References cited:
- WO-A-88/00862
- US-A- 4 368 980
- US-A- 4 428 674
- US-A- 4 606 635

## Description

The present invention relates to a method of discriminating recovered beverage containers. More specifically, the invention is concerned with a method of discriminating beverage containers for further cleaning and/or use by optically identifying the beverage residue in the recovered containers.

Systems for vending beverages such as cola, juice, beer, etc. are roughly classified into two types.

The first type is a system in which used containers are not recovered. In general, this is called as one-way system. According to this system, for instance, beverages are filled in cans at a factory, transported to an automatic vending machine, and consumed by the consumer. The can is not recovered and is thereafter discarded.

The second type is a system in which used containers are recovered and which can be called a two-way system. In this system, for instance, beverages are bottled at a factory, transported to an automatic vending machine, and consumed by the consumer. The bottles are then recovered for re-use.

The one-way system is generally convenient and has been widely employed as a preferred system. Recently, however, there is a strong voice demanding that the two-way system should be adopted, in view of the economical efficiency of reusing bottles and the effective use of vehicles for transporting the same.

However, an important question relating to the two-way system is what kind of inspection and washing should be conducted in order to obtain containers for re-use.

Hitherto, for example, recovered containers have been visually inspected by a human operator and the containers considered uncleanable have been put aside and only the good containers washed.

Such human inspection is an operation which is simple but nevertheless requires the operator to be always alert. Therefore, its mechanization has been demanded.

In contrast, another manner of inspection has been suggested, in which the beverage residue in the recovered container is withdrawn and then the residue is analyzed by means of an analyzer; see for example WO-A-88/00862. However, since the inspection using such an analyzer takes a long time and is costly, this is not efficient.

Thanks to advanced washing techniques, even if various types of foreign materials remain in the bottle, these materials do not substantially remain after washing the bottle, thereby permitting the bottle to be used again as a beverage container.

However, if tempura oil or the like remains as foreign matter in the bottle, this causes a problem by adhering to a brush of the washer and thus causing trouble in later washing.

Therefore, it has been suggested that the recovered bottles should be discriminated by determining whether they have not been utilized for any other purpose or alternatively have been so utilized. Thereafter, bottles recovered without being used for other purposes may be washed in accordance with a usual washing operation, while bottles determined to have been used for other purposes may be washed in accordance with a different washing process. Such washing system is judged to have improved efficiency and to be preferable.

When employing such a washing system, however, it becomes a problem as to how the recovered bottles are to be discriminated by determining whether or not they are bottles which have been utilized for other purposes.

On the other hand, if the beverage container has a screw cap the consumer may desire to cap the container after drinking the beverage, for the purpose of protecting the mouth of the bottle, etc. Because of this, this type of container is returned capped to the factory in a high proportion. Usually, some amount of beverage residue remains in the bottle even after the beverage is poured into a cup or the like. If the bottle is capped after drinking the beverage as above, evaporation of the beverage is inhibited. For instance, 2 cc or more of cola generally remains in a returned one-liter Coca-Cola bottle.

Even in the case of a container not having a screw cap, dried beverage matter adheres as residue to the bottom of the container.

On the other hand, where a bottle has been utilized for other purposes, there is no possibility that beverage residue remains at the bottom of the bottle.

Accordingly, recovered bottles can be discriminated as to whether they are bottles considered to have been used for other purposes or not, by detecting whether some of the beverage remains at the bottom of the recovered bottle.

According to the present invention there is provided a method of discriminating recovered beverage containers, which comprises the steps of:
selecting and storing a colour corresponding to the colour of the beverage concerned,
measuring the area of the selected colour of beverage residue in a bottom region of the container,
determining whether or not the size of the measured area of the colour representing beverage residue is within a predetermined range, and
discriminating the beverage containers in accordance with the results of the determining step.

Thus, in a method of discriminating recovered beverage containers according to the present invention, a specific colour is chosen on the basis of the colour of the beverage contained in the container. For instance, the colour of the beverage itself, or the beverage colour as seen through the container, or the colour recognized when the beverage becomes dry, etc., may be chosen.

The area of the selected colour in the container is then measured. It is then determined whether the measured colour area falls within a predetermined range or not. If the measured area of the colour is within the predetermined range, the container is judged not to have been utilized for other purposes. If on the other hand the area is outside of the range, the container is judged to have been used for other purposes.

An embodiment of the invention will now be described by way of example and with reference to the accompanying drawings, in which:-
Figure 1 is a diagram of an apparatus for carrying out the recovered bottle discriminating method according to one working example of the present invention;
Figures 2(a) and 2(b) represent a picture on the screen of a monitor TV set indicating an image of the bottle having totally no residue and a picture showing only the selected colour portion of this bottle, respectively;
Figures 3(a) and 3(b) represent a picture on the screen of a monitor TV set indicating an image of a bottle in which 2 cc of the residue remains and a picture showing only the selected colour portion of this bottle, respectively;
Figures 4(a) and 4(b) represent a picture on the screen of a monitor TV set indicating an image of a bottle in which 20 cc of the residue remains and a picture showing only the selected colour portion of this bottle, respectively; and
Figures 5(a) and 5(b) represent a picture on the screen of a monitor TV set indicating an image of a bottle in which 800 cc of the residue remains and a picture showing only the selected colour portion of this bottle, respectively.

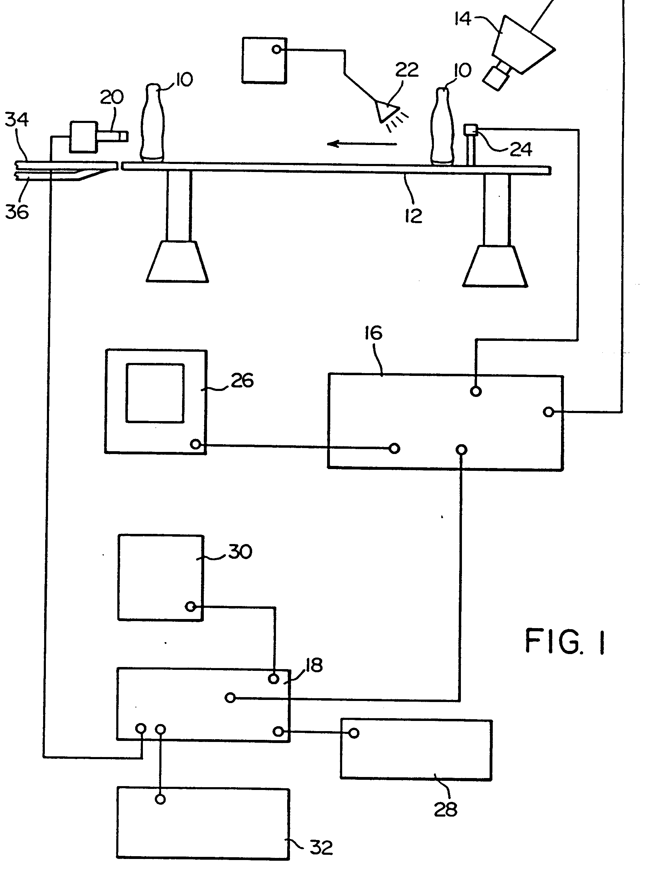

As shown in Figure 1, the discriminating apparatus is provided with a conveyor unit 12 for moving the recovered bottles 10 continuously, a camera unit 14, a colour selector 16, an information processor unit 18 and a bottle rejector 20.

The conveyor unit 12 operates to move the recovered bottles 10 continuously at a rate of 10 to 400 pcs/min., for example. An illuminator unit 22 is provided for illuminating each bottle 10 at a predetermined position on the conveyor unit 12.

On the conveyor unit 12 there is further installed a bottle sensor 24. A detection signal from the bottle sensor 24 is fed to the colour selector 16 when a bottle 10 has reached the predetermined position.

The camera unit 14 is connected to the colour selector 16. Depending on the detection signal from the bottle sensor 24, one picture is taken per bottle, for instance. In the illustrated example the camera unit 14 takes a slant picture of the bottle. When desirable, the camera unit can be so arranged as to take a picture of the bottle immediately from the top. By taking such a picture of the bottle immediately from the top, the present invention can be also applied to an opaque container. As the camera unit 14 there can be used a solid camera unit which uses a CCD having 250,000 pixels, for instance.

The colour selector 16 is further connected to a monitor TV set 26 and an image taken by the camera unit 14 is displayed on the screen of the monitor TV set 26.

Moreover, the colour selector 16 stores beforehand any colour selected depending on the colour of the beverage, the beverage colour when observed through the container, or the colour recognized when the beverage gets dry, etc. On the screen of the monitor TV set 26 there can be displayed only the selected colour portion of the picture taken by the camera unit 14 and sent to the colour selector 16. A currently available colour selector can specify a colour with sufficient precision. Where a particular colour, e.g. that of cola, is selected, a liquid having any other colour will not be displayed. Then, if the particular cola colour is not substantially present in the picture generated, the picture displayed on the screen of the monitor TV set 26 is the same as that displayed in the case of an empty bottle.

In addition, the colour selector 16 counts the number of pixels of colour on the picture taken by the camera unit 14 detecting the above selected colour and emits the counted number as an output. This pixel number corresponds to the area of the selected colour in the taken picture.

An information processor unit 18 stores a desired range of the pixel number inputted by input means in the form of a key board 28, e.g. a range of the pixel number from 60 to 60,000. If the pixel number is 60 or less this means that only a slight amount of beverage, or no beverage, remains at the bottom of the container, whereby the container is determined to have been utilized for other purposes. A pixel number of 100,000 or more indicates that the selected colour has been detected in a pixel number greater than that detected when the container is filled with the beverage. This indicates that some malfunction has occurred. These ranges can be selected according to various conditions. For instance, they can be chosen depending on the kind of camera unit used, the position relation between the camera unit and the bottles, and the shape and transparency of the bottles.

The information processor unit 18 is connected to the colour selector 16. This unit 18 receives the pixel number detected from the selected colour in the taken picture and outputted from the colour selector 16, determines whether this pixel number falls within the stored desirable range of the pixel number, and emits its result as an output.

The information processor unit 18 is further connected to a display monitor 30 and a printer 32 for displaying data of the above pixel number in a plurality of pictures for a series of the bottles 10.

The bottle rejector 20 is also connected to the information processor unit 18 and operates to sort and guide the bottles 10 to either a first track 34 or a second track 36.

Thus, a bottle 10 guided to the first track 34 will be a bottle in which a pixel number within the desired range and of the selected colour is detected in these pictures. This indicates that residue having any selected colour is present in this bottle 10 in a predetermined amount. A bottle 10 guided to the second track 36 will be a bottle in which residue of any such selected colour is not present in a predetermined amount.

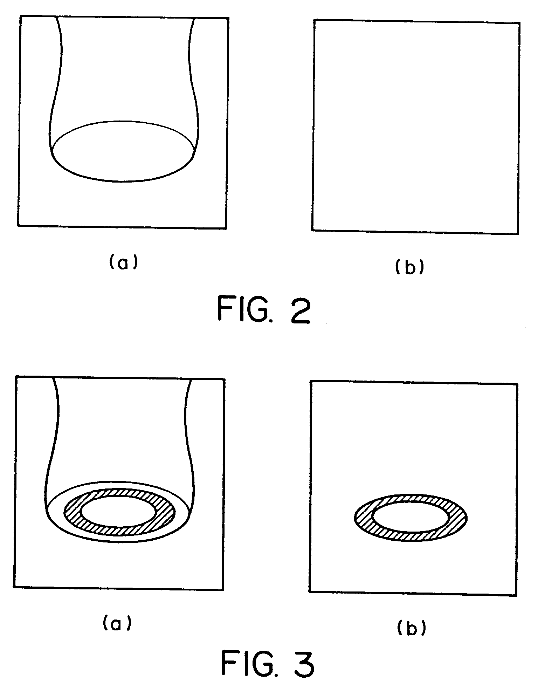

Next, by referring to Figures 2 to 5 an explanation will be made with respect to one liter size Coca-Cola bottles.

Figure 2(a) is a picture on the screen of the monitor TV set 26 indicating a normal image of such a bottle in which absolutely no residue is found. Figure 2(b) is a picture showing only the selected colour portion of this bottle. In this case, no selected colour portion is found and the pixel number detecting the selected colour is zero.

Figure 3(a) is a picture on the screen of the monitor TV set 26 indicating the image of a bottle in which a typical quantity of the residue, e.g. 2 cc, remains. Figure 3(b) is a picture showing only the portion of the selected colour of this bottle. In this case, the pixel number detecting the selected colour is 80-100, for example.

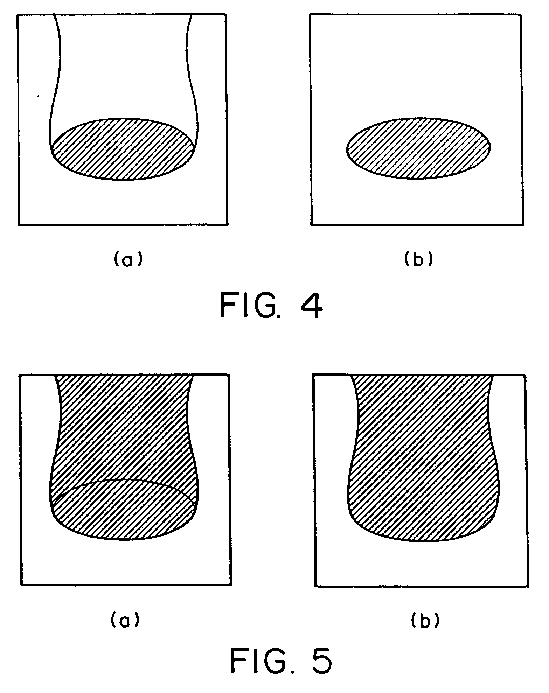

Figure 4(a) is a picture on the screen of the monitor TV set 26 indicating the image of a bottle in which the quantity of residue is relatively large, e.g. 20 cc remains. Figure 4(b) is a picture showing only the portion of the selected colour of this bottle. In this case, the pixel number detecting the selected colour is 8,000-10,000, for example.

Figure 5(a) is a picture on the screen of the monitor TV set 26 indicating the image of a bottle in which the beverage is hardly consumed and therefore, a large amount of residue remains, for example, 800 cc. In this case, the pixel number detecting the selected colour is 40,000-50,000, for example.

As shown in the above examples, where the desired range of the stored pixel number is 60-60,000, the bottle shown in Figure 2 will be guided to the second track 36 by means of the bottle rejector 20, while the bottles shown in Figures 3 to 5 will be guided to the first track 34.

Thus, it is possible to discriminate which of the bottles recovered have not been used for other purposes from those which have been used for other purposes.

Further, in the above examples, the explanation was made by considering, as an example, a container which uses a screw cap. However, the present invention can also be applied to a container not using a screw cap.

In this case, since the container is uncapped, the beverage residue remaining in the container will have dried up, and the dry beverage matter adheres to the bottom of the recovered container.

By measuring the colour and the area at the bottom of the container to which the dry beverage matter adheres it is possible to discriminate between bottles recovered without being used for other purposes and those which have been so used.

Alternatively, selection is possible by adding a small amount of water or the like to the recovered bottle, to thereby reproduce the beverage, and then measuring the colour and the area of the reproduced beverage as described above.

Further, in a situation where some recovered containers have some liquid beverage residue at the bottom and others have some dried beverage matter at the bottom, the present invention can be so applied that two or more colours are selected and any preferable range of areas occupied by these colours decided beforehand. Then the recovered bottles may be judged as not having been used for other purposes where they agree to any one of the conditions for the range of respective colour areas.

## Claims

1. A method of discriminating recovered beverage containers, which comprises the steps of:
selecting and storing a colour corresponding to the colour of the beverage concerned,
measuring the area of the selected colour of beverage residue in a bottom region of the container,
determining whether or not the size of the measured area of the colour representing beverage residue is within a predetermined range, and
discriminating the beverage containers in accordance with the results of the determining step.

2. A method according to claim 1, wherein a fixed amount of water is supplied into the container before measuring the area of the selected beverage residue colour.

3. A method according to claim 1 or 2, wherein the step of measuring the area of the selected colour of beverage residue is made by counting the number of pixels in a picture generated by a camera unit detecting the selected colour.

4. A method according to claim 3, wherein said determining step comprises the steps of:
entering and storing a pixel number count in a processor for establishing said range;
feeding the number of pixels counted of said selected colour during said measuring step to the processor; and
comparing the said counted number of pixels with the stored pixel number count in said processor.

5. A method according to any preceding claim, wherein said discriminating step further includes the step of sorting the beverage containers in accordance with the results of the determining step.

## Patentansprüche

1. Verfahren zum Differenzieren von zurückgegebenen Getränkebehältern, wobei
eine Farbe, die der Farbe des betreffenden Getränkes entspricht, ausgewählt und gespeichert wird;
die Fläche der ausgewählten Farbe des Getränkerückstands in einem Bodenbereich des Behälters gemessen wird;
festgestellt wird, ob die Größe der gemessenen Fläche der Farbe, die Getränkerückstand darstellt, innerhalb eines vorgegebenen Bereichs liegt oder nicht; und
die Getränkebehälter entsprechend den Ergebnissen der Feststellung differenziert werden.

2. Verfahren nach Anspruch 1, wobei eine feststehende Menge von Wasser in den Behälter gegeben wird, bevor die Fläche der ausgewählten Farbe des Getränkerückstands gemessen wird.

3. Verfahren nach Anspruch 1 oder 2, wobei die Messung der Fläche der ausgewählten Farbe von Getränkerückstand in der Weise gemacht wird, daß die Anzahl der Pixel in einem Bild gezählt wird, das von einer Kameraeinheit erzeugt wird, die die ausgewählte Farbe erkennt.

4. Verfahren nach Anspruch 3, wobei der Verfahrensschritt der Feststellung folgende Schritte umfaßt:
Eingeben und Speichern einer Pixel-Zahl in einem Prozessor zur Festlegung des Bereichs;
Zuführen der Zahl der während des Meß-Verfahrensschrittes gezählten Pixel der ausgewählten Farbe in den Prozessor; und
Vergleichen der gezählten Zahl von Pixeln mit der in dem Prozessor gespeicherten Zahl von Pixeln.

5. Verfahren nach einem der vorausgehenden Ansprüche, wobei der Differenzierungsschritt ferner den Verfahrensschritt des Sortierens der Getränkebehälter entsprechend den Ergebnissen des Feststellungsverfahrensschrittes enthält.

## Revendications

1. Procédé pour distinguer des récipients de boisson récupérés, comprenant les étapes consistant à :
- sélectionner et mémoriser une couleur correspondant à la couleur de la boisson concernée,
- mesurer l'aire de la couleur sélectionnée de résidu de boisson dans une région de fond du récipient,
- déterminer si la taille de l'aire mesurée de la couleur représentant le résidu de boisson est ou n'est pas dans une plage prédéterminée, et
- distinguer les récipients de boissons en fonction des résultats de l'étape de détermination.

2. Procédé selon la revendication 1, dans lequel une quantité fixée d'eau est amenée dans le récipient avant de mesurer l'aire de la couleur de résidu de boisson sélectionnée.

3. Procédé selon la revendication 1 ou 2, dans lequel l'étape de mesure de l'aire de la couleur sélectionnée de résidu de boisson est réalisée en comptant le nombre de pixels dans une image produite par une unité de caméra détectant la couleur sélectionnée.

4. Procédé selon la revendication 3, dans lequel ladite étape de détermination comprend l'étape consistant à :
- entrer et mémoriser un total de nombre de pixels dans un processeur pour établir ladite plage,
- amener le nombre de pixels comptés de ladite couleur sélectionnée durant ladite étape de mesure au processeur, et
- comparer ledit nombre compté de pixels avec le total de nombre de pixels mémorisé dans ledit processeur.

5. Procédé selon l'une quelconque des revendications précédentes, dans lequel ladite étape de distinction comprend en outre l'étape de triage des récipients de boissons en fonction du résultat de l'étape de détermination.
